# FASCICULE DE BREVET EUROPEEN

(11) **EP 1 047 403 B1**
(45) Date de publication et mention de la délivrance du brevet: **10.04.2002**
(21) Numéro de dépôt: 99947531.2
(22) Date de dépôt: 11.10.1999
(51) Int. Cl.: A61K 7/50, A61K 7/06, A61K 7/48

(54) **COMPOSITIONS COSMETIQUES CONTENANT UN TENSIOACTIF ESTER D'ALKYLPOLYGLYCOSIDE ANIONIQUE ET UN POLYMERE CATIONIQUE ET LEURS UTILISATIONS**
KOSMETISCHE ZUSAMMENSETZUNGEN ENTHALTEND EINEN ANIONISCHEN TENSID VOM TYP ALKYLPOYGLYKOSIDESTER UND EIN KATIONISCHES POLYMER SOWIE DEREN VERWENDUNGEN
COSMETIC COMPOSITIONS CONTAINING AN ANIONIC ALKYLPOLYGLYCOSIDE ESTER SURFACTANT AND A CATIONIC POLYMER AND THEIR USES

(30) Priorité: 12.11.1998 FR 9814213
(43) Date de publication de la demande: 02.11.2000
(73) Titulaire: L'OREAL, 75008 Paris (FR)
(72) Inventeur: CAUWET-MARTIN, Danièle, F-75011 Paris (FR); RESTLE, Serge, F-95390 Saint-Prix (FR)
(74) Mandataire: Dodin, Catherine
(86) Numéro de dépôt international: FR9902437
(87) Numéro de publication internationale: WO0028965

(56) Documents cités:
- EP-A- 0 510 564
- EP-A- 0 510 565
- WO-A-93/08204
- WO-A-94/27575
- DE-A- 4 302 315

## Description

La présente invention concerne de nouvelles compositions cosmétiques comprenant dans un milieu cosmétiquement acceptable au moins un tensioactif anionique de type ester d'alkylpolyglycoside carboxylique et au moins un polymère cationique ayant une densité de charge cationique supérieure ou égale à 2 meq/g.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenues dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus'couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Toutefois, et malgré les progrès réalisés récemment dans le domaine des shampooings à base de polymères cationiques et/ou de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

Cependant, les compositions classiques ont des propriétés détergentes et moussantes non satisfaisantes. De plus les matières kératiniques traitées avec ces compositions présentent le plus souvent un toucher chargé rédhibitoire.

Les tensioactifs anioniques du type ester d'alkylpolyglycoside carboxylique ont déjà été préconisés dans des compositions cosmétiques détergentes. Ils ont été décrits par exemple dans les demandes de brevet EP510565 et EP510564.

Les compositions de lavage des cheveux utilisant ces tensioactifs seuls ne conduisent pas à de bonnes propriétés cosmétiques.

L'invention a donc pour but de proposer des compositions cosmétiques présentant des propriétés cosmétiques améliorées, en particulier le démêlage et la douceur des cheveux.

Or, la demanderesse a maintenant trouvé que l'association de polymères cationiques particuliers et d'un tensioactif anionique de type ester d'alkylpolyglycoside carboxylique permettait d'atteindre ces buts.

Ces nouvelles compositions permettent de déposer une quantité plus importante de polymères cationiques sur les matières kératiniques (notamment les cheveux) qu'avec une composition contenant des tensioactifs anioniques classiques tels que les sels de lauryléthersulfates, mais sans toucher ou aspect visuel gras.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse et de discipline sans aucune sensation de toucher chargé.

L'invention a ainsi pour objet une composition cosmétique, caractérisée en ce qu'elle comprend, dans un milieu cosmétiquement acceptable, au moins un polymère cationique non cellulosique et dont la densité de charge cationique est supérieure ou égale à 2 meq/g. et au moins un tensioactif anionique de type ester d'alkylpolyglycoside carboxylique et ses sels.

Un autre objet de l'invention concerne un procédé de traitement des matières kératiniques, telles que les cheveux, caractérisé en ce qu'il consiste à appliquer sur lesdites matières des compositions cosmétiques selon l'invention.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs anioniques de type ester d'alkylpolyglycoside carboxylique peuvent avoir la structure suivante :

R₁-(O-R₂)ₜO-(G)ᵥX (I)

dans laquelle R₁ représente un radical hydrocarboné linéaire ou ramifié, saturé ou insaturé en C₆-C₃₀, de préférence un radical alkyle linéaire ou ramifié, saturé ou insaturé, comportant environ de 8 à 24 atomes de carbone, un radical alkylphényle dont le radical alkyle linéaire ou ramifié comporte environ de 8 à 24 atomes de carbone, R₂ représente un radical alkylène comportant de 2 à 4 atomes de carbone, G représente un sucre réduit comportant de 5 à 6 atomes de carbone, t désigne une valeur allant de 0 à 10 et v désigne une valeur allant de 1 à 15.
X désigne un radical comprenant au moins une fonction acide carboxylique ou ses sels. X forme une liaison ester avec un hydroxyle du sucre de préférence en position 4 ou 6.
X peut être choisi parmi les radicaux suivants :

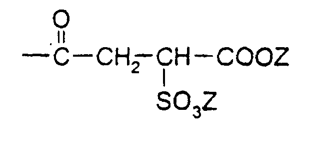

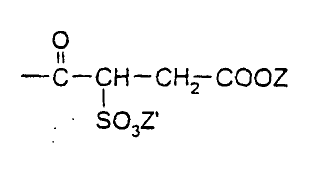

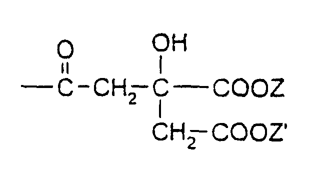

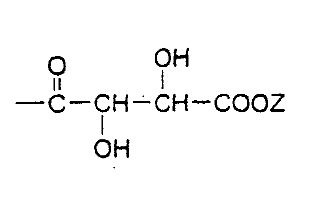

Z et Z', identiques ou différents, désignent un atome d'hydrogène, un cation minéral ou organique tel que :
un métal alcalin (par exemple Na⁺, K⁺), NH₄⁺, les ammoniums issus des aminoacides basiques tels que la lysine, l'arginine, la sarcosine, l'ornithine, la citrulline ou bien encore des amino-alcools tels que la monoéthanolamine, la diéthanolamine, la triéthanolamine, la glucamine, la N-méthyl glucamine, l'amino-3 propanediol-1,2.

Des esters d'alkylpolyglycosides préférés selon la présente invention sont des composés de formule (I) dans laquelle R₁ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation du saccharide, i.e. la valeur de v dans la formule (I), peut aller de 1 à 15. Selon l'invention, on préfère les sucres réduits contenant 80%, ou plus, de sucres dont le degré de polymérisation (S) prend une valeur allant de 1 à 4. Le degré moyen de polymérisation est plus particulièrement compris entre 1 et 2.
Les liaisons glycosidiques sont de type 1-6 ou 1-4 et de préférence 1-4.

Encore plus particulièrement, R1 désigne un radical oléyle ou un mélange de radicaux en C₈-C₁₈ dérivés du suif ou de coprah, t=0 .

Ces tensioactifs sont notamment décrits dans les demandes de brevet EP510564 et EP510565.

Parmi les tensioactifs de formule (I), on peut citer les produits commercialisés sous la dénomination EUCAROL® AEG-SS, EUCAROL® AEG-EC et EUCAROL® AEG-ET par la société CESALPINA.

Selon l'invention, le tensioactif anionique de type ester d'alkylpolyglycoside carboxylique peut représenter de 0,5 % à 30 % en poids, de préférence de 1 % à 25 % en poids, et encore plus préférentiellement de 2,5 % à 15 % en poids par rapport au poids total de la composition finale.

Les polymères cationiques utilisables selon l'invention ont une densité de charge cationique supérieure ou égale à 2 meq./g, de préférence comprise entre 2 et 8 meq./g.

La densité de charge peut être déterminée selon la méthode Kjeldahl. Elle correspond en général à un pH de l'ordre de 3 à 9.

Les polymères cationiques ayant une densité de charge cationique supérieure ou égale à 2 meq/g utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements aminé primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) Les copolymères vinylpyrrolidone-acrylate ou -méthacrylate de dialkylaminoalkyle quaternisés ou non, tels que les produits vendus sous la dénomination "Gafquat" par la Société ISP, comme par exemple Gafquat 734, 755 ou HS100 ou bien le produit dénommé "Copolymère 937". Ces polymères sont décrits en détail dans les brevets français 2 077 143 et 2 393 573.
(2) les polymères constitués de motifs pipérazinyle et de radicaux divalents alkytène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polyamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthyléne-triamine.
(6) les cyclopolymères de méthyl diallyl amine ou de diméthyl diallyl ammonium tels que les homopolymères ou copolymères comportant des motifs répondant aux formules (VI) ou (VI') : 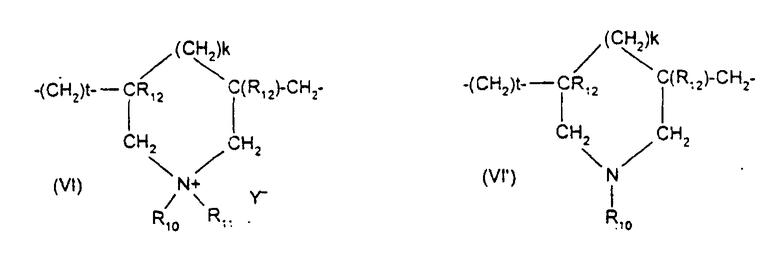 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   On peut citer par exemple l'homopolymère de chlorure de diallyldiméthylammonium commercialisé sous la dénomination "MERQUAT 100" par la société CALGON et les copolymères de chlorure de diallyldiméthylammonium et d'acrylamide commercialisés sous la dénomination "MERQUAT 550".
(7) le polymère de diammonium quaternaire contenant des motifs récurants répondant à la formule : 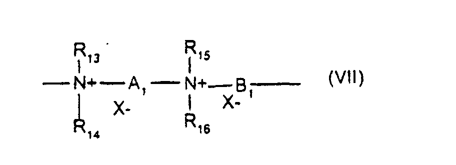 formule (VII) dans laquelle :
   R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupemant nitrile, ester, acyle, amide ou -CO-O-R17-D ou -CO-NH-R17-D où R17 est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;

   De préférence, X⁻ est un anion tel que le chlorure ou le bromure.
   Ces polymères ont une massé moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 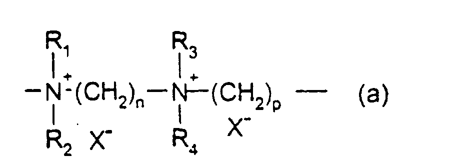 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(8) les polymères de polyammonium quaternaires constitués de motifs de formule (VIII): 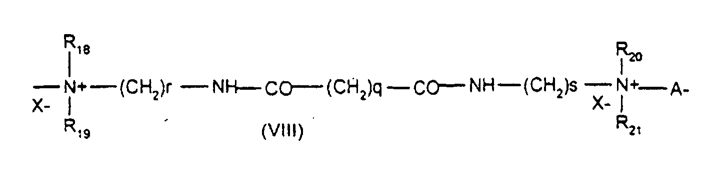 formule dans laquelle :
   R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,
   où p est égal à 0 ou à un nombre entier compris entre 1 et 6, sous réserve que R18, R19, R20 et R21 ne représentent pas simultanément un atome d'hydrogène,
   r et s, identiques ou différents; sont des nombres entiers compris entre 1 et 6,
   q est égal à 0 ou à un nombre entier compris entre 1 et 34,
   X désigne un atome d'halogène.

   A désigne un radical d'un dihalogénure ou représente de préférence -CH2-CH2-O-CH2-CH2-.
   De tels composés sont notamment décrits dans la demande de brevet EP-A-122 324.
   On peut par exemple citer parmi ceux-ci, les produits "Mirapol® A 15", "Mirapol AD1", "Mirapol AZ1" et "Mirapol 175" vendus par la société Miranol.
(9) les homopolymères ou copolymères dérivés des acides acrylique ou méthacrylique et comportant des motifs : 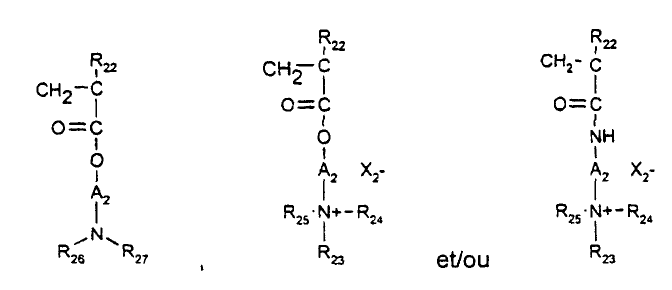 dans lesquels les groupements R22 désignent indépendamment H ou CH3,
   les groupements A1 désignent indépendamment un groupe alcoyle linéaire ou ramifié de 1 à 6 atomes de carbone ou un groupe hydroxyalcoyle de 1 à 4 atomes de carbone,
   les groupements R23, R24, R25, identiques ou différents, désignant indépendamment un groupe alcoyle de 1 à 18 atomes de carbone ouun radical benzyle,
   les groupements R26 et R27 représentent un atome d'hydrogène ou un groupement alcoyle de 1 à 6 atomes de carbone,
   X₂⁻ désigne un anion, par exemple méthosulfate ou halogénure, tel que chlorure ou bromure.

   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par HENKEL, référencé sous le nom de « POLYETHYLENEGLYCOL (15) TALLOW POLYAMINE » dans le dictionnaire CTFA.
(12) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymére dans de l'huile minérale ou dans un ester liquide. Ces dispersions sont commercialisées sous les noms de « SALCARE® SC 95 » par la Société ALLIED COLLOIDS.

D'autres polymères cationiquès utilisables dans le cadre de l'invention sont des polyalkylèneimines, en particulier des polyéthylèneimines. des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre :
- les cyclopolymères, en particulier les polymères de chlorure de diméthyldiallylammonium ou les copolymères de chlorure de diméthyldiallylammonium et d'acrylamide, vendus sous les dénominations « MERQUAT 100 » et « MERQUAT 550 » par la société CALGON.

Selon l'invention, le polymère cationique peut représenter de 0,001 % à 10 % en poids, de préférence de 0,005 % à 5 % en poids, et encore plus préférentiellement de 0,01 % à 3 % en poids, du poids total de la composition finale.

Les compositions de l'invention contiennent en outre avantageusement au moins un autre agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs additionnels convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs seis, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères' d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH2 - CHOH - SO3H
R₅ désigne un radical alkyle d'un acide carboxylique présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société HENKEL ou tel que les alkyl (C₈-C₂₀) amidoalkyl (C₁-C₆) bétaïnes en particulier la TEGOBETAINE® F 50 commercialisée par la société GOLDSCHMIDT.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

La composition de l'invention peut également contenir au moins un additif choisi parmi les épaississants, les tensioactifs, les parfums, les agents nacrants, les conservateurs, les filtres solaires, les agents tensio-actifs cationiques, les polymères anioniques ou non ioniques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones, les huiles végétales, les huiles minérales et les huiles de synthèse et tout autre additif classiquement utilisé dans le domaine cosmétique qui n'affecte pas la stabilité et les propriétés des compositions selon l'invention.

Ces additifs sont présents dans la composition selon l'invention dans des proportions pouvant aller de 0 à 50% en poids par rapport au poids total de la composition. La quantité précise de chaque additif est déterminée facilement par l'homme du métier selon sa nature et sa fonction.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols. De préférence , la composition comprend de 50 à 95 % en poids d'eau par rapport au poids total de la composition.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions selon l'invention peuvent contenir également des synergistes de mousses tels que des 1,2-alcanediols en C₁₀-C₁₈ ou des alcanolamides gras dérivés de mono ou de diéthanolamine.

Les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants.
Dans ce mode de réalisation de l'invention, les compositions comprennent une base lavante, généralement aqueuse. La base lavante peut comprendre uniquement le tensioactif anionique de type ester d'alkylpolyglycoside carboxylique ou comprendre d'autres tensioactifs additionnels.

Le ou les tensioactifs additionnels peuvent être indifféremment choisis, seuls ou en mélanges, au sein des tensioactifs anioniques, amphotères, non ioniques et cationiques tels que définis ci-dessus.

La quantité et la qualité de la base lavante sont celles suffisantes pour conférer à la composition finale un pouvoir moussant et/ou détergent satisfaisant.

Ces compositions détergentes sont de préférence moussantes et le pouvoir moussant des compositions selon l'invention, caractérisé par une hauteur de mousse, est généralement supérieur à 75 mm ; de préférence, supérieur à 100 mm mesurée selon la méthode ROSS-MILES (NF T 73-404 /ISO696) modifiée. Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin, le lavage ou le démaquillage de la peau. des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques, de gel, de lait, de crème, d'émulsion, de lotion épaissie ou de mousse et être utilisées pour la peau, les ongles, les cils, les lèvres et plus particulièrement les cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLE 1

On a réalisé une composition de shampooing conforme à l'invention :

| | A Invention | B Comparatif |
|---|---|---|
| Lauryléthersulfate de sodium (C₁₂/C₁₄) à 2,2 moles d'oxyde d'éthylène (70%MA) (MA = matière active) | - | 10 gMA |
| Disodium cocoglycoside sulfosuccinate (30%MA) | 10 gMA | ― |
| (EUCAROL AEG/SS de CESALPINA) | | |
| MERQUAT 100 de CALGON | 1 gMA | 1 gMA |
| Acide citrique qs pH | 7 | 7 |
| Eau déminéralisée qsp | 100 g | 100 g |

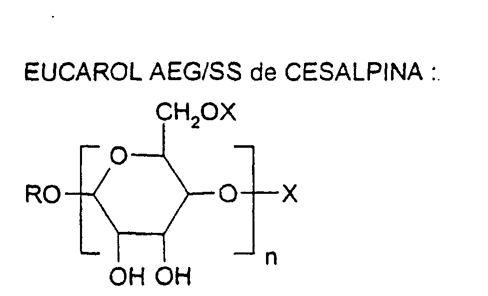

Un des radicaux X désigne hydrogène et l'autre : 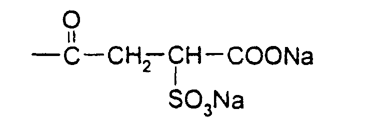 R est un radical dérivé d'alcool de coprah

MERQUAT 100 : Homopolymère de chlorure de diallyidiméthylammonium commercialisé par CALGON (densité de charge cationique environ 6,8 meq/g)

On effectue un shampooing en appliquant environ 12 g de la composition A ou de la composition B sur des cheveux décolorés préalablement mouillés. On fait mousser le shampooing puis on rince abondamment à l'eau.

Les cheveux lavés avec la composition A sont plus doux et plus faciles à démêler que ceux traités avec la composition B.

## Revendications

1. Composition cosmétique, **caractérisée par le fait qu'**elle comprend, dans un milieu cosmétiquement acceptable, au moins un polymère cationique non cellulosique ayant une densité de charge cationique supérieure ou égale à 2 meq./g et au moins un tensioactif anionique de type ester d'alkylpolyglycoside carboxylique et ses sels.

2. Composition selon la revendication 1, **caractérisée par le fait que** ledit tensioactif anionique de type ester d'alkylpolyglycoside carboxylique présente la formule (I) :
R₁-(O-R₂)ₜO-(G)ᵥX (I)
dans laquelle R₁ représente un radical hydrocarboné linéaire ou ramifié, saturé ou insaturé en C₆-C₃₀, R₂ représente un radical alkylène comportant de 2 à 4 atomes de carbone, G représente un sucre réduit comportant de 5 à 6 atomes de carbone, t désigne une valeur allant de 0 à 10 et v désigne une valeur allant de 1 à 15, X désigne un radical comprenant au moins une fonction acide carboxylique ou ses sels et X forme une liaison ester avec un hydroxyle du sucre.

3. Composition selon la revendication 2, **caractérisée par le fait que** le radical X est choisi parmi les radicaux suivants : 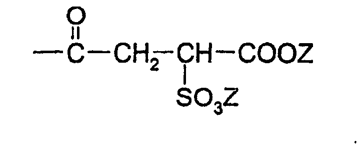 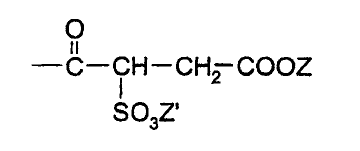 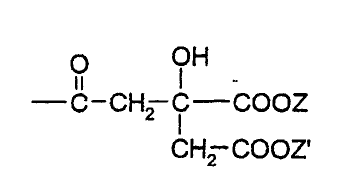 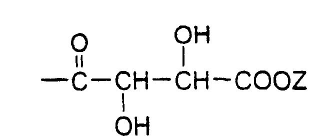 Z et Z', identiques ou différents, désignent un atome d'hydrogène, un cation minéral ou organique.

4. Composition selon l'une quelconque des revendications 2 ou 3, **caractérisée par le fait que** le radical R1 désigne un radical alkyle linéaire ou ramifié, saturé ou insaturé, comportant de 8 à 24 atomes de carbone, un radical alkylphényle dont le radical alkyle linéaire ou ramifié comporte de 8 à 24 atomes de carbone.

5. Composition selon l'une quelconque des revendications 2 à 4, **caractérisée par le fait que** le radical R1 est un radical oléyle ou un radical dérivé du coprah.

6. Composition selon l'une quelconque des revendications 2 à 5, **caractérisée par le fait que** t désigne une valeur allant de 0 à 3 et plus particulièrement encore égale à 0.

7. Composition selon l'une quelconque des revendications 2 à 6, **caractérisée par le fait que** G désigne le glucose, le fructose ou le galactose, de préférence le glucose.

8. Composition selon l'une quelconque des revendications 2 à 7, **caractérisée par le fait que** la valeur de v va de 1 à 15 et de préférence de 1 à 4.

9. Composition selon l'une quelconque des revendications 1 à 8, **caractérisée par le fait que** lesdits polymères cationiques ont une densité de charge cationique comprise entre 2 et 8 meq./g.

10. Composition selon ('une quelconque des revendications 1 à 9, **caractérisée par le fait que** lesdits polymères cationiques sont choisis parmi :
(1) Les copolymères vinylpyrrolidone-acrylate ou -méthacrylate de dialkylaminoalkyle quaternisés ou non,
(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées ;
(4) les dérivés de polyaminoamides résultant de la condensation de polyalcoylènes polyamines avec des acides polycarboxyliques suivie d'une alcoylation par des agents bifonctionnels,
(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1.
(6) les homopolymères ou copolymères comportant des motifs répondant aux formules (VI) ou (VI') : 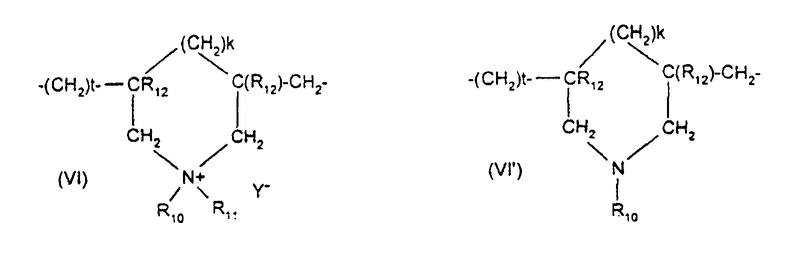 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.
(7) le polymère de diammonium quaternaire contenant des motifs récurants répondant à la formule : 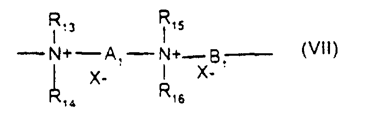 formule (VII) dans laquelle :
R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2-;
d) un groupement uréylène de formule : -NH-CO-NH- ;
(8) les polymères de polyammonium quaternaires constitués de motifs de formule (VIII): 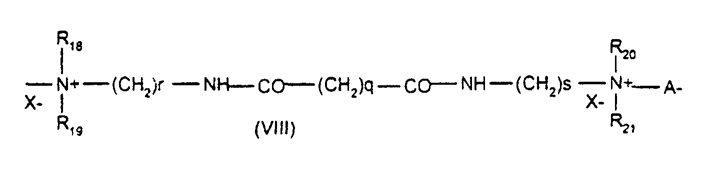 formule dans laquelle :
R18, R19, R20 et R21, identiques ou différents, représentent un atome d'hydrogène ou un radical méthyle, éthyle, propyle, β-hydroxyethyle, β-hydroxypropyle ou -CH2CH2(OCH2CH2)pOH,
où p est égal à 0 ou à un nombre entier compris entre 1 et 6, sous réserve que R18, R19, R20 et R21 ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH2-CH2-O-CH2-CH2-.
(9) les homopolymères ou copolymères dérivés des acides acrylique ou méthacrylique et comportant des motifs : 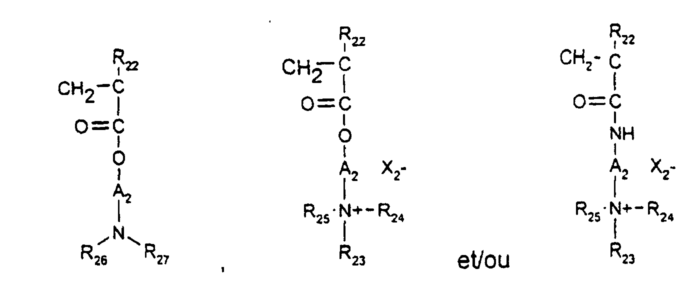 dans lesquels les groupements R22 désignent indépendamment H ou CH3,
les groupements A1 désignent indépendamment un groupe alcoyle linéaire ou ramifié de 1 à 6 atomes de carbone ou un groupe hydroxyalcoyle de 1 à 4 atomes de carbone,
les groupements R23, R24, R25, identiques ou différents, désignant indépendamment un groupe alcoyle de 1 à 18 atomes de carbone ouun radical benzyle,
les groupements R26 et R27 représentent un atome d'hydrogène ou un groupement alcoyie de 1 à 6 atomes de carbone,
X₂⁻ désigne un anion, par exemple méthosulfate ou halogénure, tel que chlorure ou bromure.
(10) Les polymères quaternaires de vinylpyrrolidone et de vinylimidazole
(11) Les polymères réticulés de sels de méthacryloyloxyalkyl(C₁-C₄) trialkyl(C₁-C₄)ammonium
(12) les polyalkylèneimines, les polymères contenant des motifs vinylpyridine ou vinylpyridinium, les condensats de polyamines et d'épichlorhydrine, les polyuréylènes quaternaires et les dérivés de la chitine.

11. Composition selon la revendication 10, **caractérisée par le fait que** lesdits polymères cationiques sont choisis parmi :
- les homopolymères de chlorure de diméthyldiallylammonium ou les copolymères de chlorure de diméthyldiallylammonium et d'acrylamide,
- les polymères qui sont constitués de motifs récurrents répondant à la formule : 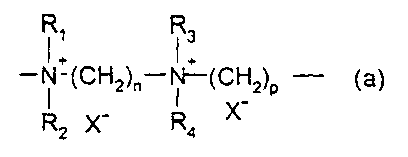
dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

12. Composition selon l'une quelconque des revendications 1 à 11, **caractérisée en ce que** le tensioactif anionique de type ester d'alkylpolyglycoside carboxylique est présent dans les compositions à une concentration comprise entre 0,5 et 30 % en poids, de préférence entre 1 et 25% en poids et plus particulièrement entre 2,5 et 15 % en poids par rapport au poids total de la composition.

13. Composition selon l'une quelconque des revendications 1 à 12, **caractérisée en ce que** ledit polymère cationique est présent à une concentration comprise entre 0,001 % et 10 % en poids par rapport au poids total de la composition, de préférence entre 0,005 % et 5 % en poids et en particulier entre 0,01 % et 3 % en poids.

14. Composition selon l'une quelconque des revendications 1 à 13, **caractérisée par le fait qu'**elle comprend en outre au moins un agent tensioactif additionnel choisi parmi les tensioactifs anioniques, cationiques, non ioniques, amphotères et leurs mélanges.

15. Compositions selon la revendication 14, **caractérisées par le fait que** le ou les agents tensioactifs additionnels sont présents à une concentration comprise entre 0,5% et 60% en poids, de préférence entre 3% et 40% en poids, et encore plus préférentiellement entre 5% et 30% en poids, par rapport au poids total de la composition.

16. Composition selon l'une quelconque des revendications 1 à 15, **caractérisée par le fait qu'**elle comprend en outre au moins un additif choisi parmi les épaississants, les parfums, les agents nacrants, les conservateurs, les filtres solaires, les agents tensioactifs cationiques, les polymères anioniques ou non ioniques, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones, les huiles végétales, les huiles minérales, les huiles de syhthèse.

17. Composition selon l'une quelconque des revendications 1 à 16, **caractérisées par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour la peau.

18. Utilisation d'une composition telle que définie dans l'une quelconque des revendications précédentes pour le lavage des matières kératiniques telles que les cheveux.

19. Procédé de traitement des matières kératiniques, telles que les cheveux, **caractérisé en ce qu'**il consiste à appliquer sur lesdites matières une composition cosmétique selon l'une des revendications 1 à 17, puis à effectuer éventuellement un rinçage à l'eau.

## Patentansprüche

1. Kosmetische Zusammensetzung, **dadurch gekennzeichnet, daß** sie in einem kosmetisch akzeptablen Medium mindestens ein kationisches Polymer, das keine Cellulose umfaßt und eine kationische Ladungsdichte von 2 meq/g oder darüber aufweist, und mindestens einen anionischen grenzflächenaktiven Stoff vom Typ der Alkylpolyglykosid-carbonsäureester und ihrer Salze enthält.

2. Zusammensetzung nach Anspruch 1, **dadurch gekennzeichnet, daß** der anionische grenzflächenaktive Stoff vom Typ der Alkylpolyglykosid-carbonsäureester der folgenden Formel (I) entspricht:
R₁-(O-R₂)ₜO-(G)ᵥX (I)
wobei die Gruppe R₁ eine geradkettige oder verzweigte, gesättigte oder ungesättigte Kohlenwasserstoffgruppe mit 6 bis 30 Kohlenstoffatomen bedeutet, die Gruppe R₂ eine Alkylengruppe mit 2 bis 4 Kohlenstoffatomen ist, die Gruppe G einen reduzierten Zucker mit 5 bis 6 Kohlenstoffatomen bedeutet, t einen Wert im Bereich von 0 bis 10 und v einen Wert im Bereich von 1 bis 15 bezeichnet und X eine Gruppe ist, die mindestens eine Carbonsäuregruppe oder ihre Salze enthält, wobei X mit einer Hydroxygruppe des Zuckers eine Esterbindung ausbildet.

3. Zusammensetzung nach Anspruch 2, **dadurch gekennzeichnet, daß** die Gruppe X unter den folgenden Gruppen ausgewählt ist: 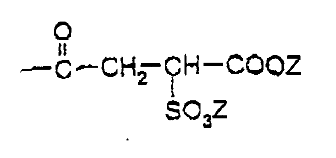 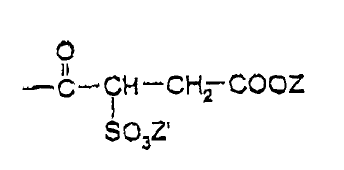 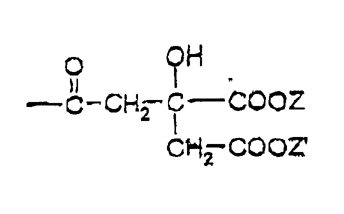 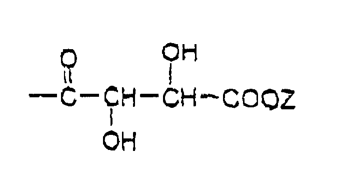 wobei Z und Z', die identisch oder voneinander verschieden sind, Wasserstoff oder ein organisches oder anorganisches Kation bedeuten.

4. Zusammensetzung nach einem der Ansprüche 2 oder 3, **dadurch gekennzeichnet, daß** die Gruppe R₁ eine geradkettige oder verzweigte, gesättigte oder ungesättigte Alkylgruppe mit 8 bis 24 Kohlenstoffatomen oder eine Alkylphenylgruppe bedeutet, deren geradkettige oder verzweigte Alkylgruppe 8 bis 24 Kohlenstoffatome aufweist.

5. Zusammensetzung nach einem der Ansprüche 2 bis 4, **dadurch gekennzeichnet, daß** die Gruppe R₁ eine Oleylgruppe oder eine von Kopra abgeleitete Gruppe ist.

6. Zusammensetzung nach einem der Ansprüche 2 bis 5, **dadurch gekennzeichnet, daß** t eine Zahl von 0 bis 3 und insbesondere 0 bedeutet.

7. Zusammensetzung nach einem der Ansprüche 2 bis 6, **dadurch gekennzeichnet, daß** G Glucose, Fructose oder Galactose und vorzugsweise Glucose bedeutet.

8. Zusammensetzung nach einem der Ansprüche 2 bis 7, **dadurch gekennzeichnet, daß** v im Bereich von 1 bis 15 und vorzugsweise 1 bis 4 liegt.

9. Zusammensetzung nach einem der Ansprüche 1 bis 8, **dadurch gekennzeichnet, daß** die kationischen Polymere eine kationische Ladungsdichte von 2 bis 8 meq/g aufweisen.

10. Zusammensetzung nach einem der Ansprüche 1 bis 9, **dadurch gekennzeichnet, daß** die kationischen Polymere ausgewählt sind unter:
(1) Copolymeren von Vinylpyrrolidon und Dialkylaminoalkylacrylat oder -methacrylat, die gegebenenfalls quaternisiert sind;
(2) Polymeren, die aus Piperazinyleinheiten und zweiwertigen Alkylen- oder Hydroxyalkylengruppen mit linearen oder verzweigten Ketten bestehen, die gegebenenfalls durch Sauerstoffatome, Schwefelatome oder Stickstoffatome oder aromatische oder heterocyclische Ringe unterbrochen sind, sowie den Oxidationsprodukten und/oder Quaternisierungsprodukten dieser Polymere;
(3) Wasserlöslichen Polyaminoamiden, die insbesondere durch Polykondensation einer Säure mit einem Polyamin hergestellt werden; die Polyaminoamide können mit einem Epihalohydrin, Diepoxid, Dianhydrid, ungesättigten Dianhydrid, zweifach ungesättigten Derivat, Bis-halohydrin, Bis-azetidinium, Bis-haloacyldiamin, Alkyl-bishalogenid oder einem Oligomer vernetzt sein, das aus der Reaktion einer bifunktionellen Verbindung resultiert, die gegenüber einem Bis-halohydrin, Bis-azetidinium, Bis-haloacyldiamin, Alkyl-bishalogenid, Epihalohydrin, Diepoxid oder zweifach ungesättigten Derivat reaktiv ist, wobei das Vernetzungsmittel in Mengenanteilen von 0,025 bis 0,35 mol pro Aminogruppe des Polyaminoamids verwendet wird; diese Polyaminoamide können alkyliert sein und eine oder mehrere tertiäre oder quartäre Aminogruppen enthalten;
(4) Polyaminoamidderivaten, die bei der Kondensation von Polyalkylenpolyaminen mit Polycarbonsäuren und anschließender Alkylierung mit bifunktionellen Mitteln resultieren;
(5) Polymeren, die durch Umsetzung eines Polyalkylenpolyamins, das zwei primäre Aminogruppen und mindestens eine sekundäre Aminogruppe aufweist, mit einer Dicarbonsäure hergestellt werden, welche unter Diglykolsäure und den gesättigten aliphatischen Dicarbonsäuren mit 3 bis 8 Kohlenstoffatomen ausgewählt ist, wobei das Molverhältnis von Polyalkylenpolyamin und Dicarbonsäure im Bereich von 0,8 : 1 bis 1,4 : 1 liegt und wobei das resultierende Polyaminoamid mit Epichlorhydrin in einem Molverhältnis Epichlorhydrin / sekundäre Aminogruppe des Polyaminoamids im Bereich von 0,5 : 1 bis 1,8 : 1 umgesetzt wird;
(6) Homopolymeren oder Copolymeren mit Einheiten der Formel (VI) oder (VI'): 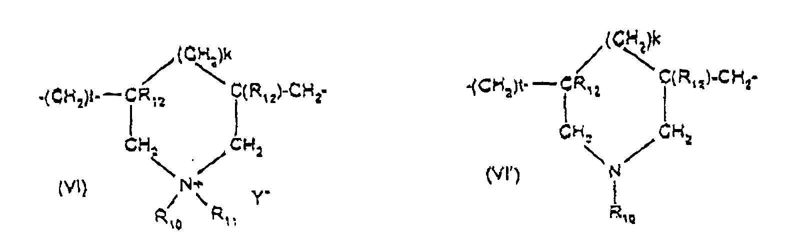 worin k und t 0 oder 1 bedeuten, wobei die Summe k + t 1 ist; R₁₂ Wasserstoff oder Methyl bedeutet; R₁₀ und R₁₁ unabhängig voneinander eine Alkylgruppe mit 1 bis 22 Kohlenstoffatomen, eine Hydroxyalkylgruppe, deren Alkylgruppe vorzugsweise 1 bis 5 Kohlenstoffatome aufweist, oder eine niedere Amidoalkylgruppe bedeuten oder R₁₀ und R₁₁ gemeinsam mit dem Stickstoffatom, an das sie gebunden sind, heterocyclische Gruppen bilden können, wie Piperidinyl oder Morpholinyl; und Y⁻ ein Anion ist, beispielsweise Bromid. Chlorid, Acetat, Borat, Citrat, Tartrat, Bisulfat, Bisulfit, Sulfat oder Phosphat;
(7) Quartären Diammoniumpolymeren mit wiederkehrenden Einheiten der folgenden Formel; 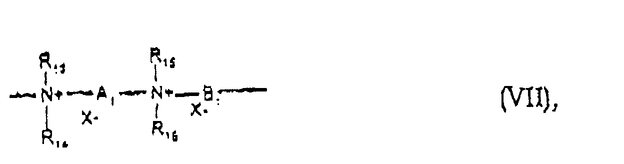 wobei in der Formel (VII) bedeuten:
- die Gruppen R₁₃, R₁₄, R₁₅ und R₁₆, die identisch oder voneinander verschieden sind, aliphatische, alicyclische oder arylaliphatische Gruppen mit 1 bis 20 Kohlenstoffatomen oder niedere hydroxyalkylaliphatische Gruppen, oder R₁₃, R₁₄, R₁₅ und R₁₆ bilden gemeinsam oder unabhängig voneinander mit den Stickstoffatomen, an die sie gebunden sind, Heterocylen, die gegebenenfalls ein weiteres Heteroatom, das von Stickstoff verschieden ist, enthalten, oder R₁₃, R₁₄, R₁₅ und R₁₆ bedeuten eine geradkettige oder verzweigte C₁₋₆-Alkylgruppe, die substituiert ist mit einer Gruppe Nitril, Ester, Acyl, Amid oder -CO-O-R₁₇-D oder -CO-NH-R₁₇D, worin R₁₇ eine Alkylengruppe bedeutet und D eine quartäre Ammonimgruppe ist;
- A₁ und B₁ Polymethylengruppen mit 2 bis 20 Kohlenstoffatomen, die geradkettig oder verzweigt, gesättigt oder ungesättigt vorliegen können und an die Hauptkette gebunden oder in der Hauptkette einen oder mehrere aromatische Ringe oder ein oder mehrere Sauerstoffatome oder Schwefelatome oder die folgenden Gruppen enthalten können: Sulfoxid, Sulfon, Disulfid, Amino, Alkylamino, Hydroxy, quartäre Ammoniumgruppen, Ureido, Amid oder Ester und
- X⁻ ein Anion, das von einer anorganischen oder organischen Säure abgeleitet ist;
- wobei A₁, R₁₃ und R₁₅ gemeinsam mit den beiden Stickstoffatomen, an die sie gebunden sind, einen Piperazinring bilden können;
- wenn A₁ eine geradkettige oder verzweigte, gesättigte oder ungesättigte Alkylen- oder Hydroxyalkylengruppe bedeutet, kann B₁ ferner eine (CH₂)ₙ-CO-D-OC-(CH₂)ₙ-Gruppe bedeuten, worin D bedeutet;
a) einen Glykolrest der Formel: -O-Z-O-, worin Z eine geradkettige oder verzweigte Kohlenwasserstoffgruppe oder eine Gruppe der folgenden beiden Formeln bedeutet:
-(CH₂-CH₂-O)ₓ-CH₂CH₂-
-[CH₂-CH(CH₃)-O]_{y}-CH₂-CH(CH₃)-
worin x und y eine ganze Zahl von 1 bis 4 bedeuten und einen definierten und einzigen Polymerisationsgrad darstellen oder eine beliebige Zahl von 1 bis 4 bedeuten und einen mittleren Polymerisationsgrad darstellen;
b) einen bis-sekundären Diaminrest, beispielsweise ein Piperazinderivat;
c) einen bis-primären Diaminrest der Formel: -NH-Y-NH, worin Y eine geradkettige oder verzweigte Kohlenwasserstoffgruppe oder die zweiwertige Gruppe -CH₂-CH₂-S-S-CH₂-CH₂- bedeutet; oder
d) eine Ureylengruppe der Formel: -NH-CO-NH-;
(8) Quartären Polyammoniumpolymeren, die aus Einheiten der Formel (VIII) bestehen: 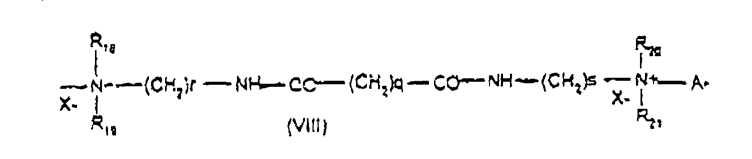 worm bedeuten:
- R₁₈, R₁₉, R₂₀ und R₂₁, die identisch oder voneinander verschieden sind, Wasserstoff, Methyl, Ethyl, Propyl, β-Hydroxyethyl, β-Hydroxypropyl oder -CH₂CH₂(OCH₂CH₂)ₚOH, wobei p 0 oder eine ganze Zahl von 1 bis 6 bedeutet, mit der Maßgabe, daß R₁₈, R₁₉, R₂₀ und R₂₁ nicht gleichzeitig Wasserstoff bedeuten,
- r und s, die identisch oder voneinander verschieden sind, ganze Zahlen von 1 bis 6,
- q 0 oder eine ganze Zahl von 1 bis 34,
- X ein Halogenatom,
- A eine Dihalogenidgruppe oder vorzugsweise die Gruppe
-CH₂-CH₂-O-CH₂-CH₂-.
(9) Homopolymeren oder Copolymeren, die von Acryl- oder Methacrylsäure abgeleitet sind und die folgenden Einheiten enthalten: 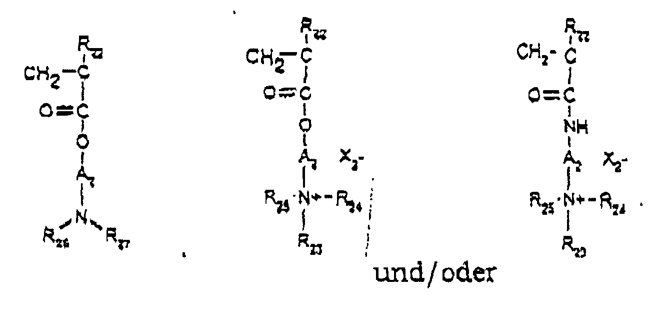 worin;
- die Gruppen R₂₂ unabhängig voneinander H oder CH₃ bedeuten,
- die Gruppen A₂ unabhängig cine geradkettige oder verzweigte Alkylgruppe mit 1 bis 6 Kohlenstoffatomen oder eine Hydroxyalkylgruppe mit 1 bis 4 Kohlenstoffatomen bedeuten,
- die Gruppen R₂₃, R₂₄ und R₂₅, die identisch oder voneinander verschieden sind, unabhängig voneinander eine Alkylgruppe mit 1 bis 18 Kohlenstoffatomen oder eine Benzylgruppe bedeuten,
- die Gruppen R₂₆ und R₂₇ ein Wasserstoffatom oder eine Alkylgruppe mit 1 bis 6 Kohlenstoffatomen bedeuten,
- X₂⁻ ein Anion bedeutet, beispielsweise Methosulfat oder ein Halogenid, wie Chlorid oder Bromid;
(10) quartären Polymeren von Vinylpyrrolidon und Vinylimidazol;
(11) Vernetzten Polymeren von Methacryloyloxy(C₁₋₄)alkyltri(C₁₋₄)alkyl-ammoniumsalzen; und
(12) Polyalkyleniminen, Polymeren, die Vinylpyridin- oder Vinylpyridinium-Einheiten enthalten, Kondensaten von Polyaminen und Epichlorhydrin, quartären Polyureylenen und Chitinderivaten.

11. Zusammensetzung nach Anspruch 10, **dadurch gekennzeichnet, daß** die kationischen Polymere ausgewählt sind unter:
- Homopolymeren von Dimethyldiallylammoniumchlorid oder Copolymeren von Dimethyldiallylammoniumchlorid und Acrylamid;
- Polymeren, die aus wiederkehrenden Einheiten der folgenden Formel bestehen: 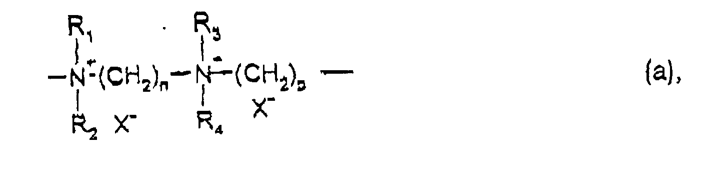
worin die Gruppen R₁, R₂, R₃ und R4, die identisch oder voneinander verschieden sind, eine Alkyl- oder Hydroxyalkylgruppe mit etwa 1 bis 4 Kohlenstoffatomen sind, n und p ganze Zahlen im Bereich von etwa 2 bis 20 bedeuten und X⁻ ein Anion ist, das von einer anorganischen oder organischen Säure abgeleitet ist.

12. Zusammensetzung nach einem der Ansprüche 1 bis 11, **dadurch gekennzeichnet, daß** der anionische grenzflächenaktive Stoff vom Typ Alkylpolyglykosid-carbonsäureester in den Zusammensetzungen in einer Konzentration von 0,5 bis 30 Gew.-%, vorzugsweise 1 bis 25 Gew.-% und insbesondere 2,5 bis 15 Gew.-%, bezogen auf das Gesamtgewicht der Zusammensetzung, vorliegt.

13. Zusammensetzung nach einem der Ansprüche 1 bis 12, **dadurch gekennzeichnet, daß** das kationische Polymer in einer Konzentration von 0,001 bis 10 Gew.-%, bezogen auf das Gesamtgewicht der Zusammensetzung, vorzugsweise 0,005 bis 5 Gew.-% und insbesondere 0,01 bis 3 Gew.-% vorliegt.

14. Zusammensetzung nach einem der Ansprüche 1 bis 13, **dadurch gekennzeichnet, daß** sie ferner mindestens einen zusätzlichen grenzflächenaktiven Stoff enthält, der unter den anionischen, kationischen, nichtionischen oder amphoteren grenzflächenaktiven Stoffen und deren Gemischen ausgewählt ist.

15. Zusammensetzung nach Anspruch 14, **dadurch gekennzeichnet, daß** der oder die zusätzlichen grenzflächenaktiven Stoffe in einer Konzentration von 0,5 bis 60 Gew.-%, vorzugsweise 3 bis 40 Gew.-% und noch bevorzugter 5 bis 30 Gew.-%, bezogen auf das Gesamtgewicht der Zusammensetzung, vorliegt.

16. Zusammensetzung nach einem der Ansprüche 1 bis 15, **dadurch gekennzeichnet, daß** sie ferner mindestens einen Zusatzstoff enthält, der unter den Verdickungsmitteln, Parfums, Perlglanzpigmenten, Konservierungsmitteln, Sonnenschutzfiltern, kationischen grenzflächenaktiven Stoffen, anionischen oder nichtionischen Polymeren, Proteinen, Proteinhydrolysaten, Ceramiden, Pseudoceramiden, Fettsäuren mit geraden oder verzweigten C₁₆₋₄₀-Ketten, beispielsweise 18-Methyleicosansäure, Hydroxysäuren, Vitaminen, Panthenol, Siliconen, pflanzlichen Ölen, Mineralölen und synthetischen Ölen ausgewählt ist.

17. Zusammensetzung nach einem der Ansprüche 1 bis 16, **dadurch gekennzeichnet, daß** sie als Haarwaschmittel, Haarpflegemittel, Zusammensetzung für Dauerwellen, Entkräuselungen, Färbungen oder Entfärbungen der Haare, Zusammensetzung, die ausgespült wird und zwischen den beiden Schritten einer Dauerwelle oder Entkräuselung aufgetragen wird, oder reinige Zusammensetzung für die Haut vorliegt.

18. Verwendung einer Zusammensetzung nach einem der vorhergehenden Ansprüche zum Waschen von Keratinsubstanzen, wie dem Haar.

19. Verfahren zur Behandlung von Keratinsubstanzen, wie dem Haar, **dadurch gekennzeichnet, daß** auf die Keratinsubstanzen eine kosmetische Zusammensetzung nach einem der Ansprüche 1 bis 17 aufgetragen und gegebenenfalls anschließend mit Wasser gespült wird.

## Claims

1. Cosmetic composition, **characterized in that** it comprises, in a cosmetically acceptable medium, at least one noncellulosic cationic polymer having a cationic charge density greater than or equal to 2 meq/g and at least one anionic surfactant of the alkylpolyglycoside carboxylic ester type and its salts.

2. Composition according to Claim 1, **characterized in that** the said anionic surfactant of the alkylpolyglycoside carboxylic ester type has the formula (I) :
R₁-(O-R₂)ₜO-(G)ᵥX (I)
in which R₁ represents a saturated or unsaturated, linear or branched C₆-C₃₀ hydrocarbon radical, R₂ represents an alkylene radical comprising from 2 to 4 carbon atoms, G represents a reduced sugar comprising from 5 to 6 carbon atoms, t denotes a value ranging from 0 to 10 and v denotes a value ranging from 1 to 15, X denotes a radical comprising at least one carboxylic acid function or its salts and x forms an ester bond with a hydroxyl of the sugar.

3. composition according to Claim 2, **characterized in that** the X radical is chosen from the following radicals: 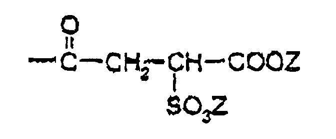 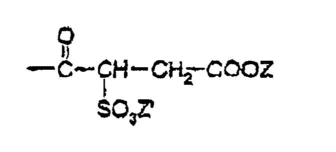 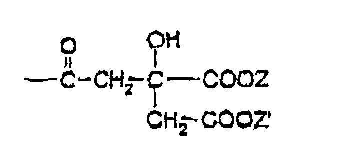 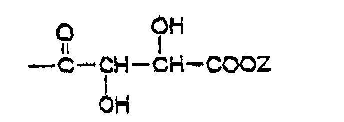 Z and Z', which are identical or different, denote a hydrogen atom, or an inorganic or organic cation.

4. Composition according to either of Claims 2 and 3, **characterized in that** the R₁ radical denotes a saturated or unsaturated, linear or branched alkyl radical comprising from 8 to 24 carbon atoms, an alkylphenyl radical in which the linear or branched alkyl radical comprises from 8 to 24 carbon atoms.

5. Composition according to any one of Claims 2 to 4, **characterized in that** the R₁ radical is an oleyl radical or a radical derived from copra.

6. Composition according to any one of Claims 2 to 5, **characterized in that** t denotes a value ranging from 0 to 3 and still more particularly equal to 0.

7. Composition according to any one of Claims 2 to 6, **characterized in that** G denotes glucose, fructose or galactose, preferably glucose.

8. Composition according to any one of Claims 2 to 7, **characterized in that** the value of v ranges from 1 to 15 and preferably from 1 to 4.

9. Composition according to any one of claims 1 to 8, **characterised in that** the said cationic polymers have a cationic charge density of between 2 and 8 meq/g.

10. Composition according to any one of Claims 1 to 9, **characterized in that** the said cationic polymers are chosen from:
(1) vinylpyrrolidone-dialkylaminoalkyl acrylate or methacrylate copolymers, quaternized or otherwise,
(2) polymers consisting of piperazinyl units and of alkylene or hydroxyalkylene divalent radicals with straight or branched chains, optionally interrupted by oxygen, sulphur or nitrogen atoms or by aromatic or heterocyclic rings, as well as the oxidation and/or quaternization products of these polymers;
(3) water-soluble polyaminoamides prepared in particular by polycondensation of an acid compound with a polyamine; these polyaminoamides may be crosslinked with an epihalohydrin, a diepoxide, a dianhydride, an unsaturated dianhydride, a diunsaturated derivative, a bishalohydrin, a bisazetidinium, a bishaloacyldiamine, an alkyl bishalide or else with an oligomer resulting from the reaction of a difunctional compound which is reactive towards a bishalohydrin, a bisazetidinium, a bishaloacyldiamine, an alkyl bishalide, an epihalohydrin, a diepoxide or a diunsaturated derivative; the crosslinking agent being employed in proportions ranging from 0.025 to 0.35 mol per amine group of the polyaminoamide; these polyaminoamides may be alkylated or, if they include one or more tertiary amine functional groups, quaternized;
(4) polyaminoamide derivatives resulting from the condensation of polyalkylenepolyamines with polycarboxylic acids, followed by an alkylation with difunctional agents.
(5) polymers obtained by reaction of a polyalkylenepolyamine containing two primary amine groups and at least one secondary amine group with a dicarboxylic acid chosen from diglycolic acid and saturated aliphatic dicarboxylic acids containing from 3 to 8 carbon atoms. The molar ratio of the polyalkylenepolyamine to the dicarboxylic acid being between 0.8 : 1 and 1.4 : 1; the polyaminoamide resulting therefrom being made to react with epichlorohydrin in a molar ratio of epichlorohydrin relative to the secondary amine group of the polyaminoamide of between 0.5 : 1 and 1.8 : 1.
(6) the homopolymers or copolymers comprising units corresponding to the formulae (VI) or (VI'): 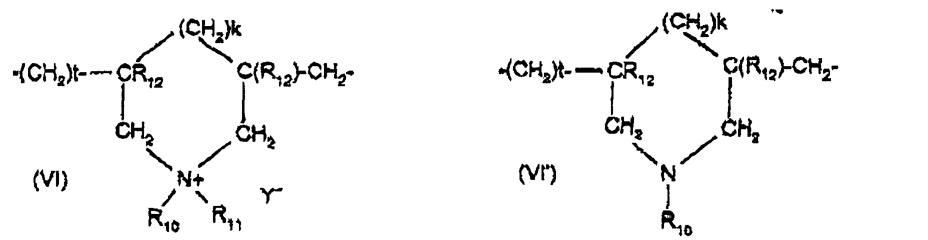 in which formulae k and t are equal to 0 or 1, the sum k + t being equal to 1; R₁₂ denotes a hydrogen atom or a methyl radical; R₁₀ and R₁₁, independently of each other, denote an alkyl group containing from 1 to 22 carbon atoms, a hydroxalkyl group in which the alkyl group preferably has 1 to 5 carbon atoms, or a lower amidoalkyl group or R₁₀ and R₁₁ may denote, jointly with the nitrogen atom to which they are attached, heterocyclic groups such as piperidinyl or morpholinyl; Y⁻ is an anion such as bromide, chloride, acetate, borate, citrate, tartrate, bisulphate, bipulphite, sulphate or phosphate.
(7) the quaternary diammonium polymer containing repeat units corresponding to the formula: 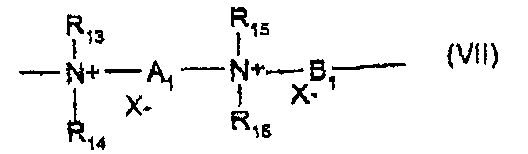 formula (VII) in which:
R₁₃, R₁₄, R₁₅ and R₁₆, which are identical or different, represent aliphatic, aliayalic or arylaliphatic radicals containing from 1 to 20 carbon atoms or lower hydroxyalkyl aliphatic radicals, or else R₁₃, R₁₄, R₁₅ and R₁₆, together or separately, form, with the nitrogen atoms to which they are attached, heterocyclic rings optionally containing a second heteroatom other than nitrogen, or else R₁₃, R₁₄, R₁₅ and R₁₆ denote a linear or branched C₁-C₆ alkyl radical substituted by a nitrile, ester, acyl, amide or -CO-O-R₁₇-D or -CO-NH-R₁₇-D group where R₁₇ is an alkylene and D a quaternary ammonium group;
A₁ and B₁ represent polymethylene groups containing from 2 to 20 carbon atoms which may be linear or branched, saturated or unsaturated and which may contain, bonded to or inserted into the main chain, one or more aromatic rings, or one or more oxygen or sulphur atoms or sulphoxide, sulphone, disulphide, amino, alkylamino, hydroxyl, quaternary ammonium, ureido, amide or ester groups, and
X⁻ denotes an anion derived from an inorganic or organic acid;
A₁, R₁₃ and R₁₅, with the two nitrogen atoms to which they are attached, may form a piperazine ring; in addition if A₁ denotes a saturated or unsaturated, linear or branched alkylene or hydroxyalkylene radical, B₁ may also denote a group (CH₂)ₙ-CO-D-OC-(CH₂)ₙ-
in which D denotes:
a) a glycol residue of formula: -O-Z-O-, where Z denotes a linear or branched hydrocarbon radical or a group corresponding to one of the following formulae:
-(CH₂-CH₂-O)ₓ-CH₂-CH₂-
-[CH₂-CH(CH₃)-O]_{y}-CH₂-CH(CH₃)-
where x and y denote an integer from 1 to 4, representing a defined and unique degree of polymerization or any number from 1 to 4 representing a mean degree of polymerization;
b) a disecondary diamine residue such as a piperazine derivative;
c) a diprimary diamine residue of formula: -NH-Y-NH-, where Y denotes a linear or branched hydrocarbon radical or else the divalent radical
-CH₂-CH₂-S-S-CH₂-CH₂-;
d) a ureylene group of formula; -NH-CO-NH-;
(8) quaternary palyammonium polymers consisting of units of formula (VIII) : 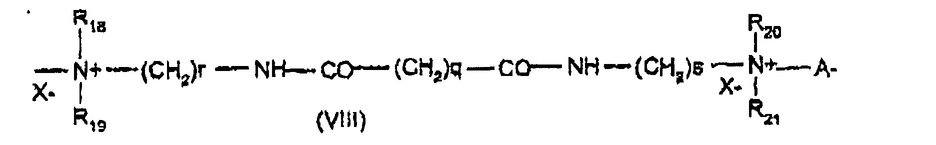 in which formula:
R₁₈, R₁₉, R₂₀ and R₂₁, which are identical or different, denote a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, provided that R₁₈, R₁₉, R₂₀ and R₂₁ do not simultaneously denote a hydrogen atom,
r and s, which are identical or different, are integers between 1 and 6,
q is equal to 0 or to an integer between 1 and 34,
X denotes a halogen atom,
A denotes a radical of a dihalide or preferably represents -CH₂-CH₂-O-CH₂-CH₂-.
(9) the homopolymers or copolymers derived from methacrylic or acrylic acids and comprising units: 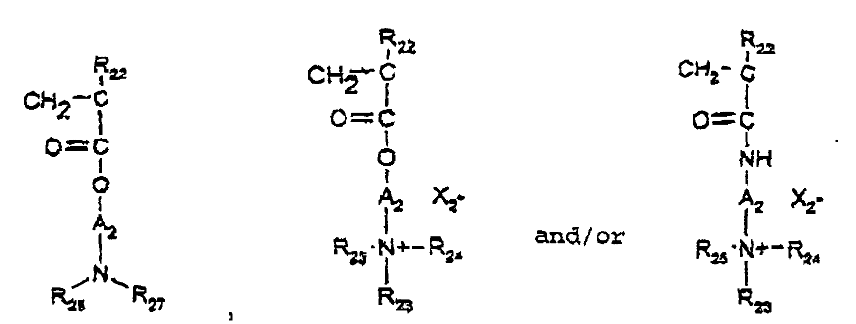 in which the groups R₂₂ denote independently H or CH₃,
the groups A₁ denote independently a linear or branched alkyl group of 1 to 6 carbon atoms or a hydroxyalkyl group of 1 to 4 carbon atoms,
the groups R₂₃, R₂₄, R₂₅, which are identical or different, independently denoting an alkyl group of 1 to 18 carbon atoms or a benzyl radical,
the groups R₂₆ and R₂₇ represent a hydrogen atom or an alkyl group of 1 to 6 carbon atoms,
X₂⁻ denotes an anion, for example methosulphate or halide, such as chloride or bromide.
(10) quaternary polymers of vinylpyrrolidone and vinylimidazole
(11) crosslinked polymers of salts of methacryloyloxy(C₁-C₄)alkyl(tri(C₁-C₄ alkyl))ammonium
(12) polyalkyleneimines, polymers containing vinylpyridine or vinylpyridinium units, condensates of polyamines and epichlorohydrin, quaternary polyureylenes and chitin derivatives.

11. Composition according to Claim 10, **characterized in that** the said cationic polymers are chosen from:
- dimethyldiallylammonium chloride homopolymers or copolymers of dimethyldiallylammonium chloride and acrylamide,
- polymers which consist of recurring units corresponding to the formula: 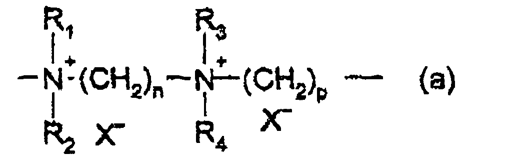 in which R₁, R₂, R₃ and R₄, which are identical or different, denote an alkyl or hydroxyalkyl radical having from 1 to 4 carbon atoms approximately, n and p are integers varying from 2 to 20 approximately and X⁻ is an anion derived from an inorganic or organic acid.

12. Composition according to any one of Claims 1 to 11, **characterized in that** the anionic surfactant of the alkylpolyglycoside carboxylic ester type is present in the compositions at a concentration of between 0.5 and 30% by weight, preferably between 1 and 25% by weight and more particularly between 2.5 and 15% by weight relative to the total weight of the composition.

13. Composition according to any one of Claims 1 to 12, **characterized in that** the said cationic polymer is present at a concentration of between 0.001% and 10% by weight relative to the total weight of the composition, preferably between 0.005% and 5% by weight and in particular between 0.01% and 3% by weight.

14. Composition according to any one of Claims 1 to 13, **characterized in that** it comprises, in addition, at least one additional surfactant chosen from anionic, cationic, nonionic and amphoteric surfactants and mixtures thereof.

15. Composition according to Claim 14, **characterized in that** the additional surfactant(s) are present at a concentration of between 0.5% and 60% by weight, preferably between 3% and 40% by weight and still more preferably between 5% and 30% by weight, relative to the total weight of the composition.

16. Composition according to any one of Claims 1 to 15, **characterized in that** it comprises, in addition, at least one additive chosen from thickeners, perfumes, pearlescent agents, preservatives, sunscreens, cationic surfactants, anionic or nonionic polymers, proteins, protein hydrolysates, ceramides, pseudoceramides, linear or branched C₁₆-C₄₀ chain fatty acids such as 18-methyleicosanoic acid, hydroxy acids, vitamins, panthenol, silicones, vegetable oils, mineral oils and synthetic oils.

17. Composition according to any one of Claims 1 to 16, **characterized in that** it is provided in the form of a shampoo, a hair conditioner, a composition for permanent waving, hair straightening, dyeing or bleaching the hair, a rinse-off composition to be applied between the two stages of a permanent waving or hair straightening treatment, or a cleansing composition for the skin.

18. Use of a composition as defined in any one of the preceding claims for washing keratinous materials such as the hair.

19. Method of treating keratinous materials, such as the hair, **characterized in that** it consists in applying to the said materials a cosmetic composition according to one of claims 1 to 17, and then in optionally carrying out a rinsing with water.
